Europäisches Patentamt

European Patent Office

Office européen des brevets

(11) Veröffentlichungsnummer: **0 024 601**

**A1**

## (12) EUROPÄISCHE PATENTANMELDUNG

(21) Anmeldenummer: 80104682.2

(22) Anmeldetag: 08.08.80

(51) Int. Cl.³: **A 61 M 1/03**
A 61 M 5/16, B 01 D 35/00
B 01 D 29/26

(30) Priorität: 22.08.79 DE 7923865 U

(43) Veröffentlichungstag der Anmeldung:
11.03.81 Patentblatt 81/10

(84) Benannte Vertragsstaaten:
AT BE CH DE FR GB IT LI LU NL SE

(71) Anmelder: Biotest-Serum-Institut GmbH
Flughafenstrasse 4
D-6000 Frankfurt-Niederrad(DE)

(72) Erfinder: Walker, Wolfram H.,Dr. Dipl.-Chem.
Leipziger Strasse 12
D-6074 Rödemark(DE)

(72) Erfinder: Gänshirt, Karl-Heinz Dr. Dipl.-Chem.
August-Bebel-Strasse 34
D-6072 Dreieich(DE)

(74) Vertreter: Beil, Walter, Dr. et al,
BEIL, WOLFF & BEIL Rechtsanwälte Adelonstrasse 58
D-6230 Frankfurt am Main 80(DE)

(54) Mikrofiltrationsgerät zur Filtration von Koageln und Mikroaggregaten von Blut.

(57) Bei diesem Mikrofiltrationsgerät weist die Filterkammer (6) mit zunehmender Filtereffektivität mindestens zwei kaskadenförmig hintereinandergeschaltete gleich große, zunehmende oder abnehmende Filterelemente (3,4) auf, die in Sandwichform aufeinanderliegend oder durch eine Abstandshalterung voneinander getrennt, stehend oder hängend angeordnet sind und deren Filterflächen zylindrisch, kegelstumpfartig oder in Faltenform ausgebildet sind und aus Oberflächenfiltern oder Tiefenfiltern oder Kombinationen davon bestehen, wobei das Gerät ein Füllvolumen von weniger als 150 ml besitzt. Das Gerät dient der Filtration von zur Transfusion bestimmtem gelagertem Blut und Blutbestandteilen.

Fig. 2

Unsere Nr. 22 959                    Pr/br

Biotest-Serum-Institut GmbH
6000 Frankfurt-Niederrad

Mikrofiltrationsgerät zur Filtration von
Koageln und Mikroaggregaten von Blut

Die Erfindung betrifft ein Mikrofiltrationsgerät zur Filtration von Koageln und Mikroaggregaten von Blut. Bei der Lagerung von Blut entstehen abhängig von verschiedenen Parametern wie Blutqualität, Blutstabilisatorlösung und Lagerungsart Blut-Mikrokoagel in der Größenordnung von 10 µm bis über 300 µm.

Die Koagel bestehen hauptsächlich aus Thrombozyten, Fibrinogen sowie anderen zellulären und plasmatischen Blutbestandteilen sowie Zellfragmenten.

Bei der Transfusion von gelagertem Blut und Blutbestandteilen besteht die Gefahr, daß diese Koagel insbesondere bei Massivtransfusion in den Kreislauf eingeschwemmt werden und primär in der Lunge als dem "physiologischen" Filter zurückgehalten werden. Hierbei können diese Koagel zu dem Effekt des sog. "Wet Lung Syndrom" oder der "Transfusionslunge", d.h. zu einer Verstopfung der Lungenkapillaren beitragen.

Aus diesem Grunde wurden seit Jahren bei Transfusionsbestecken sog. Transfusionsfilter eingesetzt.

Es handelt sich um reine Sieb- oder Maschenfilter mit mittleren Porengrößen von 170 bis 310 µm. So beschreibt DIN 58 360 ein solches Transfusionsbesteck und legt gleichzeitig die Oberfläche mit 10 cm² sowie eine Maschenweite von mindestens 310 µm fest.

In den letzten Jahren wurden nun Spezialfilter entwickelt, sogenannte Mikroaggregat- oder Mikrokoagelfilter, die in der Lage sind, Blutkoagel bis in den Größenbereich von 10 µm aus dem Blut herauszufiltrieren. Bei diesen Mikrokoagelfiltern unterscheidet man zwischen Siebfilter oder Tiefenfilter. Bei Tiefenfiltern beruht der Filtrationseffekt auf einer unselektiven Adsorption von Koageln durch eine Schicht mehr oder weniger dicht gepackter Fasern bzw. durch einen porösen Schaum. Siebfilter dagegen arbeiten mit einem Filter definierter Maschenweite. Es gibt auch Kombinationen dieser Filterprinzipien.

An Mikrokoagelfilter werden ganz allgemein von dem Kliniker eine Reihe von Anforderungen gestellt. Hierzu gehört eine hohe Filtereffektivität des Gerätes, eine hohe Filterkapazität, eine hohe Durchflußrate, ein kleines Füllvolumen sowie eine günstige Handhabung des Gerätes.

Außerdem darf ein solches Filter selbst bei Drücken bis zu 400 mm Hg und bei der Verabreichung mehrerer Konserven keinen ungünstigen Einfluß auf die Blutparameter, wie Hämolyse etc., zeigen. Außerdem muß es die
Handhabung, d.h. Umstecken auf mehrere Blutkonserven
sowie Drucktransfusion ohne Schaden an Filter und
Gehäuse überstehen. Es muß andererseits als Einmalgerät
möglichst preisgünstig konstruiert sein.

Im DE-GM 76 05 700 wird ein Mikrokoagelfilter beschrieben, bei dem das Gerät aus einem Gehäuse besteht, in
das bis zu 5 Filter, vorzugsweise mindestens 4 Filter
in Form von Sieben, eingebracht werden, die eine Kaskadenanordnung zeigen, dh., die Siebe werden von dem
zu filtrierenden Blut nacheinander durchflossen, wobei
das nachfolgende Sieb eine höhere Filterleistung zeigt
als das vorausgegangene Sieb. Dadurch kann erreicht
werden, daß bei relativ kleiner Filteroberfläche
und bei selektiver Beladung der Siebflächen ein günstiger
Filtrationseffekt vorliegt.
Das in dem obengenannten GM beschriebene Gerät zeigt
außerdem eine Tropfkammer, die direkt mit der Filtrationskammer hermetisch verbunden ist. Bei dem gesamten Gerät handelt es sich um ein sterilisierbares
Einmalgerät. Die Siebe zeigen bei abnehmender Porengröße eine abnehmende Filterfläche, sind vorzugsweise
in Kegelstumpfform ausgebildet und bestehen vorzugsweise
aus reinen Siebfiltern, deren kleinste Maschenweite
10 µm beträgt. Das Füllvolumen des Gerätes beträgt
ca. 150 ml. Ein solches Gerät ist z.B. in der Lage, ca. 5 bis 10 ACD-Vollblutkonserven, die ca. 2 Wochen lagerten, innerhalb von 30 Minuten unter Schwerkraftbedingungen zu filtrieren.

Trotz dieser relativ günstigen Eigenschaften erwies sich dieses Gerät für gewisse Zwecke als zu groß und unhandlich.

Aufgabe der Erfindung war es deshalb, ein Gerät bereitzustellen, das den Anforderungen an Mikrokoagelfilter entspricht, jedoch bei ausreichender Effektivität und Kapazität ein kleines Volumen aufweist.

Es wurde nun gefunden, daß die Anforderungen an Mikrokoagelfilter teilweise auch erreicht werden können, wenn nur mindestens 2 Filterelemente unterschiedlicher Effektivität hintereinander geschaltet werden. Bei einer optimalen Abstimmung von Filterfläche, Filterleistung und Geometrie des Filters kann auch bei kleinem Füllvolumen von ca. 30 bis 50 ml trotzdem eine ausreichende Effektivität und Kapazität erreicht werden. Dies gilt insbesondere dann, wenn das letzte Sieb eine mittlere Maschenweite von 20 bis 100 µm zeigt. Ein solches Gerät mit der Kombination 150 und 50 µm ist z.B. noch in der Lage, ca. 2 bis 5 ACD-Vollblutkonserven, die 2 Wochen lagerten, innerhalb von 15 bis 30 Minuten unter Schwerkraftbedingungen zu filtrieren. Die Filterflächen dieser Filterelemente können, abhängig von Filterleistung, gleich groß, aber auch zunehmend oder abnehmend vorliegen. Sie können sowohl in zylindrischer als auch in kegelstumpfartiger Form, vorzugsweise ineinandergesteckt, vorliegen.

Die Variation der Filterfläche kann z.B. auch durch sternförmige Faltung oder durch Einstülpung des Filterelements in W- oder M-Form erfolgen. Die Filterelemente können sowohl in stehender als auch in hängender Form angeordnet sein.

Hierbei spielt es keine Rolle, ob die Filterflächen aus reinen Sieben oder Netzen bestehen oder aber ob Flächen mit Tiefenfilterwirkung, wie z.B. Vliese oder Schäume, eingesetzt werden. Es ist z.B. auch eine Kombination von Sieben und Vliesen einsetzbar. Weiter ist es möglich, zwei oder mehrere Filterelemente, vorzugsweise drei, in Sandwichform aufeinander zu legen, wobei die Siebflächen unmittelbaren Kontakt haben oder aber über ein Grobsieb oder einen Abstandshalter voneinander getrennt sind.

Vorzugsweise wird ein solches Gerät bis auf die Filterflächen aus Spritzgußteilen hergestellt. Die Siebflächen müssen einzeln oder als Sandwich hermetisch mit der Filtrationskammer verbunden sein. Vorzugsweise besitzt ein solches Gerät eine integrierte Tropfkammer.

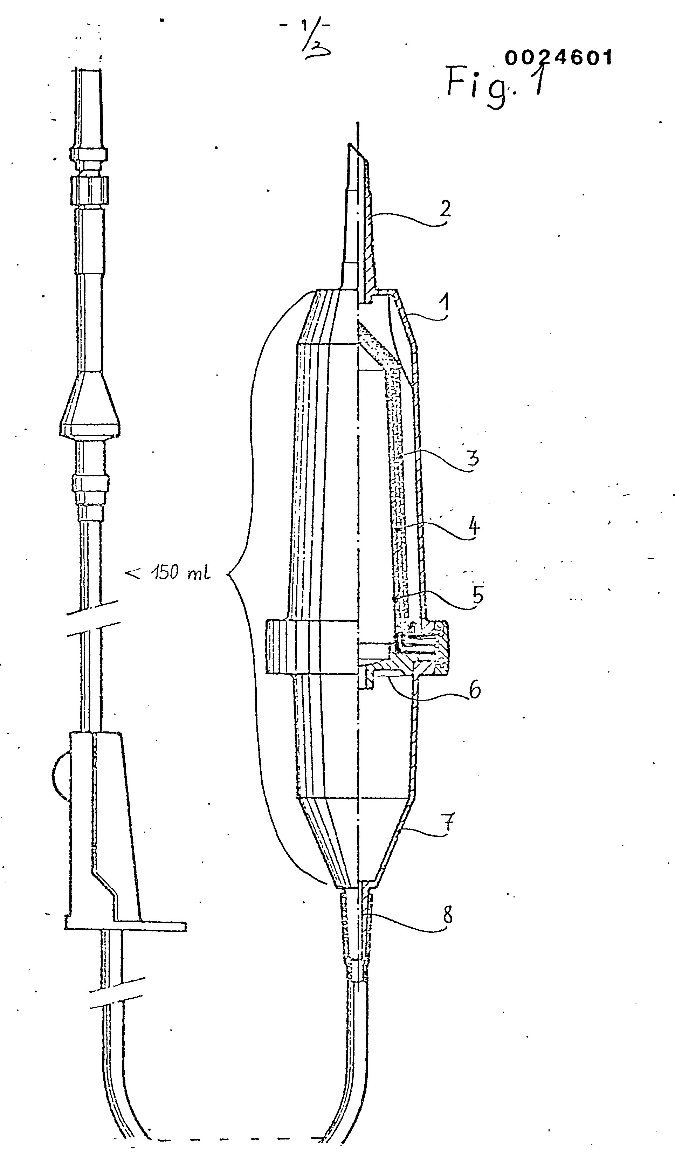

Fig. 1 zeigt eine typische Ausführungsform für ein solches Gerät. Es besteht aus Gehäuse (1) mit Einstichdorn (2) und 3 Filterelementen (3), (4), (5), die in diesem Fall zylindrisch stehend angeordnet sind. Gehäuse, die 3 Filterelemente, Auslaufteil der Filterkammer (6) sowie Tropfkammer (7) mit Auslauf (8) sind hermetisch über einen Dichtungsring hintereinander verbunden. Die Dichtung muß absolut bakterien- und gasdicht sein und kann sowohl durch Verschweißung, Verklebung oder auch Umspritzung erreicht werden. Ein solches Gerät ist auch für Drucktransfusionen geeignet.

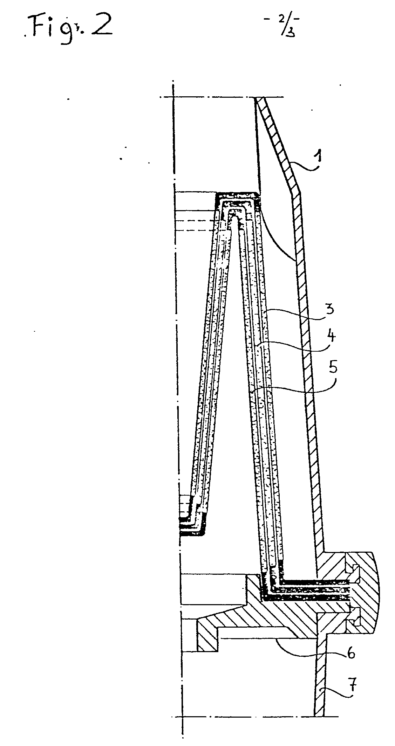

Fig. 2 zeigt einen Längsschnitt durch einen Abschnitt einer weiteren Ausführungsform eines Filtrationsgerätes, worin die Filterelemente (3), (4), (5) als M-Form ausgebildete Filterflächen aufweisen und ohne Abstandshalter stehend angeordnet sind. Die Ziffern (1), (6) und (7) haben die gleiche Bedeutung wie in Fig. 1.

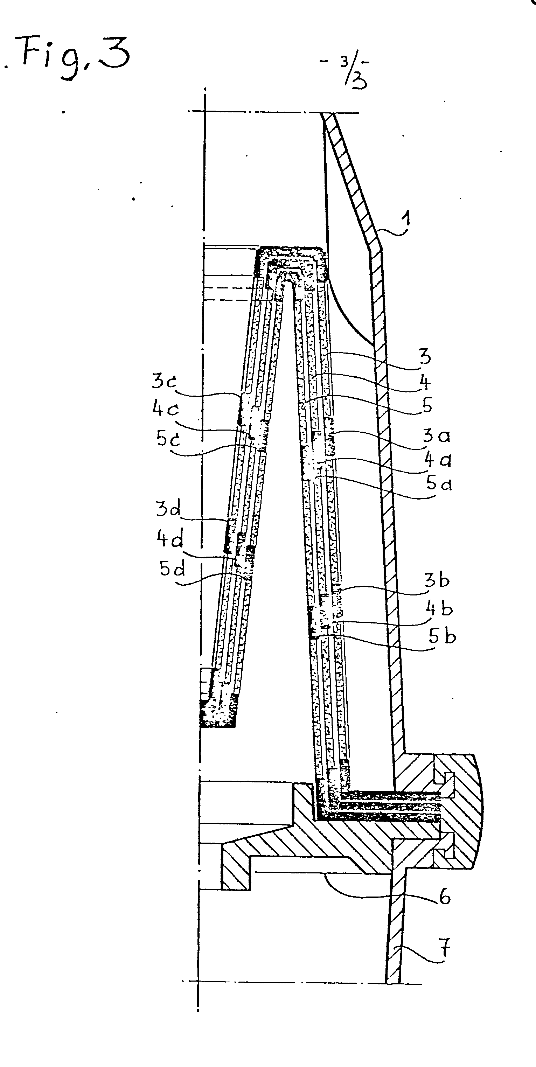

Fig. 3 zeigt einen Längsschnitt durch einen Abschnitt einer weiteren Ausführungsform eines Filtrationsgerätes, worin die Filterelemente (3), (4), (5) ähnlich wie in Fig. 2 als M-Form ausgebildete Filterflächen aufweisen und stehend angeordnet sind, jedoch mit Abstandshaltern (3a, b, c, d) (4a, b, c, d) und (5a, b, c,d) versehen sind.
Die Ziffern (1), (6) und (7) haben ebenfalls die gleiche Bedeutung wie in Fig. 1.

Bei der Anwendung von Nylonsieben als Filterflächen können die mittleren Maschenweiten dieser Siebe zwischen 400 und 10 µm, vorzugsweise 150, 50 und 10 µm bestehen, die Faserdurchmesser dieser Siebe zwischen 5 und 100 µm liegen.

0024601

- 7 -

<u>P a t e n t a n s p r ü c h e:</u>

1. Mikrofiltrationsgerät zur Filtration von Koageln und Mikroaggregaten von Blut und Blutbestandteilen, bestehend aus einem Gehäuse (1) mit Filterkammer (6), Einstichdorn (2) und mit zunehmender Filtereffektivität kaskadenförmig hintereinandergeschalteten Filterelementen und einer gegebenenfalls integrierten Tropfkammer (7) mit Auslauf (8), wobei alle Teile des Gerätes gas- und keimdicht verschlossen sind, <u>dadurch gekennzeichnet</u>, daß die Filterkammer (6) mindestens 2 gleich große, zunehmende oder abnehmende Filterelemente (3,4), die in Sandwichform aufeinanderliegend oder durch eine Abstandshalterung voneinander getrennt, stehend oder hängend angeordnet sind und deren Filterflächen zylindrisch, kegelstumpfartig oder in Faltenform ausgebildet sind und aus Oberflächenfiltern oder Tiefenfiltern oder Kombinationen davon bestehen, aufweist und das Gerät ein Füllvolumen von weniger als 150 ml besitzt.

2. Gerät nach Anspruch 1, dadurch gekennzeichnet, daß es 3 Filterelemente (3,4,5) aufweist.

3. Gerät nach Anspruch 1 oder 2, dadurch gekennzeichnet, daß die Filterelemente ineinandergesteckt angeordnet sind.

4. Gerät nach Anspruch 1 bis 3, dadurch gekennzeichnet, daß die Oberflächenfilter aus Netzen, Geweben oder Sieben bestehen.

5. Gerät nach Anspruch 1 bis 3, dadurch gekennzeichnet, daß die Tiefenfilter aus Vliesen oder Schäumen bestehen.

6. Gerät nach einem der Ansprüche 1 bis 4, dadurch gekennzeichnet, daß die Filterflächen aus Gewebesieben der Faserdurchmesser von 100 bis 5 µm und die Kaskadenanordnung aus Sieben der mittleren Maschenweiten von 400 bis 10 µm bestehen.

7. Gerät nach Anspruch 6, dadurch gekennzeichnet, daß die Kaskadenanordnung aus 2 Sieben (3,4) von 150 und 50 oder aus 3 Sieben (3,4,5) von 150, 50 und 10 µm besteht.

Fig. 1

1/3

< 150 ml

Fig. 2

- 2/3 -

0024601

Fig. 3

- 3/3 -

0024601

## Europäisches Patentamt
## EUROPÄISCHER RECHERCHENBERICHT

### EINSCHLÄGIGE DOKUMENTE

| Kategorie | Kennzeichnung des Dokuments mit Angabe. soweit erforderlich, der maßgeblichen Teile | betrifft Anspruch |
|---|---|---|
| | US - A - 3 765 536 (ROSENBERG) <br><br> * Figuren 1-3; Spalte 2, Zeilen 54-68; Spalte 3, Zeilen 1-47 und letzter Absatz; Spalte 4; Spalte 5, Zeilen 1-48; Spalte 6, Zeilen 23-42; 58-68; Spalte 7, Zeilen 1-22 * <br><br> -- | 1,2,4, 6 |
| | FR - A - 2 305 196 (BIOTEST-SERUM) <br><br> * Figur 1; Seite 1, Zeilen 1-4, 25-35; Seite 2, Zeilen 1,2, 11-26 und letzter Absatz; Seite 3 * <br><br> -- | 1,3,4 |
| | US - A - 2 644 586 (CUTTER) <br><br> * Figuren 1-3; Spalte 1, Zeilen 1-16; Spalte 2, Zeilen 21-55; Spalte 3, Zeilen 1-3 * <br><br> -- | 1,3,4 |
| | US - A - 4 157 965 (RAIBLE) <br><br> * Figuren 1,2,4,6,7; Spalte 2, Zeilen 19-23, 55-68; Spalte 3, Zeilen 11-29; Spalte 4, Zeilen 10-63 * <br><br> -- | 1,3-5, 7 |
| | FR - A - 2 407 013 (MEDICAL INC.) <br><br> * Figuren 1,6,7; Seite 1, Absatz 1 und Zeilen 32-35; Seite 3, Zeilen 29-38; Seite 4, Zeilen 16-32; Seite 6, Zeilen 11-16; Seite 13, Zeilen 30-37; Seite 14, Zeilen 1-7; Seite 15, Zeilen 32-37; Seite 16, Zeilen 1,2; Seite 17, Zeilen 1-34 * <br><br> ---- | 1,4,6 |

**KLASSIFIKATION DER ANMELDUNG (Int Cl³)**

A 61 M 1/03
A 61 M 5/16
B 01 D 35/00
29/26

**RECHERCHIERTE SACHGEBIETE (Int Cl³)**

A 61 M
B 01 D

**KATEGORIE DER GENANNTEN DOKUMENTE**

X: von besonderer Bedeutung
A: technologischer Hintergrund
O: nichtschriftliche Offenbarung
P. Zwischenliteratur
T. der Erfindung zugrunde liegende Theorien oder Grundsatze
E: kollidierende Anmeldung
D· in der Anmeldung angefuhrtes Dokument
L: aus andern Grunden angefuhrtes Dokument
&· Mitglied der gleichen Patent-familie. übereinstimmendes Dokument

Der vorliegende Recherchenbericht wurde für alle Patentansprüche erstellt

| Recherchenort | Abschlußdatum der Recherche | Prufer |
|---|---|---|
| Den Haag | 21-11-1980 | MAROSCIA |

EPA form 1503.1  06.78